# EUROPEAN PATENT APPLICATION

(11) **EP 3 342 404 A1**
(43) Date of publication of application: **04.07.2018**
(21) Application number: 17210906.8
(22) Date of filing: 28.12.2017
(51) Int. Cl.: A61K 9/48, A61K 45/06

(54) **PHARMACEUTICAL COMPOSITIONS OF DICLOFENAC AND ELETRIPTAN**

(30) Priority: 29.12.2016 TR 201619983
(71) Applicant: Sanovel Ilac Sanayi ve Ticaret A.S., 34460 Istanbul (TR)
(72) Inventor: TÜRKYILMAZ, Ali, 34460 Istanbul (TR); ZENGINER, Sibel, 34460 Istanbul (TR); DEDEOGLU, Yavuz, 34460 Istanbul (TR)
(74) Representative: Sevinç, Erkan

(57) **Abstract**

The present invention relates to a pharmaceutical composition comprising diclofenac or a pharmaceutically acceptable salt thereof in combination with eletriptan or a pharmaceutically acceptable salt thereof and at least one pharmaceutically acceptable excipient.

## Description

### Field of Invention

The present invention relates to a pharmaceutical composition comprising diclofenac or a pharmaceutically acceptable salt thereof in combination with eletriptan or a pharmaceutically acceptable salt thereof and at least one pharmaceutically acceptable excipient.

### The background of the invention

Diclofenac is a widely used medicine for relieving pain and inflammation. It is available in the sodium, potassium, epolamine and diethylamine salt forms in numerous dosage forms and it belongs to a group of medicines called 'non-steroidal anti-inflammatory drugs' (NSAIDs). Nonsteroidal anti-inflammatory drugs (NSAIDs) are a structurally diverse group of agents with analgesic, antipyretic and anti-inflammatory properties. The primary mechanism of action of these drugs is believed to be the inhibition of enzymes of the cyclo-oxygenase (COX) family, which are involved in the biosynthesis of prostaglandins.

Owing to its poor solubility in water, Diclofenac is normally used in salt form; the salts of Diclofenac customarily used are those of sodium, potassium or other alkali and alkaline earth metals, together with salts of organic nature, such as the salts of basic amino acids, such as lysine, arginine and omithine, or other pharmacologically acceptable organic bases which have the ability to render the resulting salt soluble in water.

Diclofenac molecule is disclosed in US3558690.

Its chemical name is 2-[(2,6-Dichlorophenyl)amino]benzeneacetic acid and its chemical structure is shown in the Formula 1.

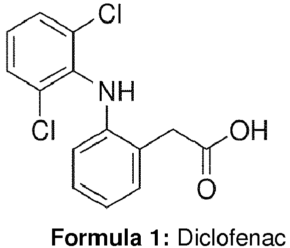

5-HT is a key mediator in the pathogenesis of migraine. 5-HT1-receptor agonists, commonly known as the 'triptans', are the mainstay for acute treatment of migraine headaches.

Generally, effective triptans may be selected from a group consisting of, but not limited to sumatriptan, rizatriptan, eletriptan, naratriptan, zolmitriptan, frovatriptan, almotriptan, and functional analogs thereof, wherein the functional analogs have essentially the same biological activity.

Eletriptan (trade name Relpax®, used in the form of eletriptan hydrobromide) is a second generation triptan drug intended for treatment of migraine headaches. Its chemical name is 3-[[(2R)-1-methylpyrrolidin-2-yl]methyl]-5-(2-phenylsulfonylethyl)-1 H-indole and its chemical structure is shown in the Formula 2.

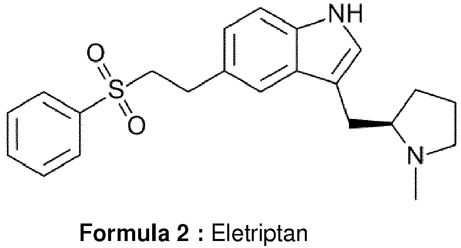

Eletriptan molecule and the use for the treatment of migraine is first disclosed in the patent application WO9206973.

Migraines are likely due to local cranial vasodilatation and/or to the release of sensory neuropeptides (vasoactive intestinal peptide, substance P and calcitonin gene-related peptide) through nerve endings in the trigeminal system. The therapeutic activity of eletriptan for the treatment of migraine headache is thought to be due to the agonist effects at the 5-HT1B/1D receptors on intracranial blood vessels (including the arterio-venous anastomoses) and sensory nerves of the trigeminal system which result in cranial vessel constriction and inhibition of pro-inflammatory neuropeptide release.

In prior art, patent application EP2306998 discloses a combination comprising a 5-HT1 agonist and a NSAID, but no patent application specifically discloses a pharmaceutical composition comprising diclofenac in combination with eletriptan in oral administration as tablet or capsule dosage form and there is no combination of diclofenac and eletriptan in the market for the treatment of migraine headache.

Thus, there is a need in the art for a pharmaceutical composition or a dosage form comprising a combination of diclofenac and eletriptan to achieve improved migrain headache treatment. However, while combining two active agents, some problems occur such as physicochemical incompatibility problems. For this reason, it is very essential to choose active agents and excipients used in the formulation which comprises two active agents.

### Detailed description of the invention

The main object of this present invention is to provide a pharmaceutical composition comprising diclofenac or a pharmaceutically acceptable salt thereof in combination with eletriptan or a pharmaceutically acceptable salt thereof and at least one pharmaceutically acceptable excipient.

According to one embodiment of the present invention, diclofenac or a pharmaceutically acceptable salt thereof is present in an amount of between %5 and %40 by weight of total formulation, preferably between %10 and %30, more preferably between %10 and %20, eletriptan or a pharmaceutically acceptable salt thereof is present in an amount of between %5 and %40 by weight of total formulation, preferably between %10 and %30, more preferably between %10 and %20.

According to another embodiment of the present invention, the weight ratio of diclofenac or acceptable salt thereof to eletriptan or acceptable salt thereof is between 0.2 and 5.0, preferably between 0.3 and 3.0, more preferably between 0.5 and 2.0.

Drugs of different action mechanisms can be combined. It is possible, however, to state that a combination of drugs having different action mechanisms, but showing actions on similar targets, will have absolutely positive effects.

While combining more than one molecule in one dosage form is increasing the patients' quality of life, improving patient adherence, many challenges also occur such as the physicochemical compatibility between the different active agents and/or between the active agents and the excipients used; and the therapeutical compatibility between the two active agents regarding their pharmacokinetic and/or pharmaceutical properties in order that the posology of the combined formulation allows to obtain safe and efficient plasma levels of both pharmacological agents. It is well known that drugs even used in the same therapeutic area cannot always be combined effectively in a dosage form.

When eletriptan and diclofenac are used together in one dosage form, improved therapeutic effect on migraine headache are obtained with reduced risk of adverse reactions relative to higher dose monotherapy.

Thus, choice of active agents and excipients is essential. Both active agents should be stable with all excipients and each other. To achieve the stability of both diclofenac and eletriptan, selection of excipients and the ratio of them is very essential. The percentage of disintegrant is effective to achieve desired dissolution profile with stable formulation. In this present invention, a pharmaceutical dosage form comprising diclofenac in combination with eletriptan has been developed with safe and effective dissolution profiles for each drug molecule and an easy process.

According to one embodiment of the present invention, the pharmaceutical composition is in the form of tablet, mini tablet, bilayer tablet, trilayer tablet, multilayer tablet, capsule, tablet in tablet, an inlay tablet, injectable preparate, suspension, syrup, sachet, ointment, cream or gel.

According to this embodiment, preferably the dosage form is in the form of a capsule.

According to another embodiment of the present invention, the pharmaceutically acceptable excipient is selected from a group comprising disintegrants, fillers, binders, lubricants, glidants or mixtures thereof.

Suitable disintegrants are selected from a group comprising cross-linked carboxymethyl cellulose sodium (croscarmellose sodium), starch, crospovidone (cross-linked polyvinil pyrrolidone), poloxomer, low-substituted hydroxypropyl cellulose, pregelatinized starch, sodium carboxymethyl cellulose, calcium carboxymethyl cellulose, carboxymethyl cellulose, docusate sodium, guar gum, , polyacryline potassium, sodium alginate, corn starch, sodium starch glycolate, alginic acid, alginates, ion-exchange resins, magnesium aluminium silica, sodium dodesyl sulphate, sodium glycine carbonate, sodium lauryl sulphate or mixtures thereof.

In a preferred embodiment, the disintegrant is cross-linked carboxymethyl cellulose sodium (croscarmellose sodium).

In one embodiment, the disintegrant is present in an amount of between 0.1 % and 10%, preferably between 0.5% and 10%, more preferably between 1% and 10% by weight of total formulation.

The percentage of disintegrant is effective to achieve desired dissolution profile with stable formulation. Especially croscarmellose sodium (cross-linked carboxymethyl cellulose sodium) is preferred as a disintegrant.

Suitable fillers are selected from a group comprising microcrystalline cellulose, lactose monohydrate, lactose, mannitol, spray-dried mannitol, starch, dextrose, sucrose, fructose, maltose, sorbitol, xylitol, inositol, kaolin, inorganic salts, calcium salts, polysaccharides, dicalcium phosphate, sodium chloride, dextrates, lactitol, maltodextrin, sucrose-maltodextrin mixture, trehalose, sodium carbonate, sodium bicarbonate, calcium carbonate, polyols, dextrose, maltitol or mixtures thereof.

In a preferred embodiment, the fillers are microcrystalline cellulose and lactose monohydrate.

Suitable binders are selected from the group comprising polyvinylpyrrolidone (povidone), copovidone, copolyvidone, carnauba wax, hydroxypropyl methyl cellulose, pullulan, polymethacrylate, glyceryl behenate, hydroxypropyl cellulose, carboxymethyl cellulos, methyl cellulose, hydroxyethyl cellulose, sodium carboxymethyl cellulose, carboxymethyl cellulose calcium, ethyl cellulose, polymetacrylates, polyethylene oxide, polyvinyl alcohol, polycarbophil, polyvinyl acetate and its copolymers, gelatin, starch, pregelatinized starch, xanthan gum, guar gum, alginate, carrageen, kollagen, agar, pectin, hyaluronic acid, carbomer, cellulose acetate phthalate, hydroxypropyl starch, hydroxyethyl methyl cellulose, polaxomer, polyethylene glycol, sugars, glycose syrups, natural gums, tragacanth gum, polyacrylamide, aluminum hydroxide, benthonite, laponite, setostearyl alcohol, polyoxyethylene-alkyl ethers, acacia mucilage, polydextrose or mixtures thereof.

In a preferred embodiment, the binder is povidone (polyvinylpyrrolidone), preferably povidone K30.

Suitable lubricants are selected from a group comprising magnesium stearate, glyceryl behenate, calcium stearate, zinc stearate, talc, waxes, boric acid, hydrogenated vegetable oil, sodium chlorate, magnesium lauryl sulfate, sodium oleate, sodium acetate, sodium benzoate, polyethylene glycol, stearic acid, fatty acid, fumaric acid, glyseryl palmito sulphate, sodium stearyl fumarate, sodium lauryl sulphate or mixtures thereof.

In a preferred embodiment, the lubricant is magnesium stearate.

Suitable glidants are selected from a group comprising colloidal silicon dioxide, talc, aluminium silicate or mixtures thereof.

In a preferred embodiment, the glidant is colloidal silicon dioxide.

Suitable salts of eletriptan are selected from a group comprising acetate, aspartate, benzoate, besylate, bicarbonate/carbonate, bisulphate/sulphate, hemisulfate, borate, camsylate, citrate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleat, malonate, mesylate, methylsulphate, naphthylate, 2-napsylate, nicotinate, nitrate, orotate, oxalate, palmitat, pamoate, phosphate/hydrogen phosphate/dihydrogen phosphate, saccharate, stearate, succinate, tartrate, tosylat and trifluoroacetate, aluminium, arginine, benzathine, calcium, choline, diethylamine, diolamine, glycine, lysine, magnesium, meglumine, lamine, potassium, sodium, tromethamine and zinc salts.

Suitable salts of diclofenac are selected from a group comprising potassium, sodium, resinate, calcium, hydrochloride, epolamine and diethylamine.

In a preferred embodiment, the pharmaceutically acceptable salt of eletriptan is eletriptan hydrobromide and the pharmaceutically acceptable salt of diclofenac is diclofenac potassium.

According to one preferred embodiment, the pharmaceutical composition comprises:
a. 5.00 to 40.00 % diclofenac or a pharmaceutically acceptable salt thereof
b. 5.00 to 40.00 % eletriptan or a pharmaceutically acceptable salt thereof
c. 5.00 to 50.00 % microcrystalline cellulose
d. 5.00 to 50.00 % lactose monohydrate
e. 0.1 to 5.00 % povidone (polyvinylpyrrolidone)
f. 0.1 to 10.00 % croscarmellose sodium (cross-linked carboxymethyl cellulose sodium)
g. 0.1 to 10.00 % colloidal silicon dioxide
h. 0.1 to 10.00 % magnesium stearate
by weight of the total formulation

According to one preferred embodiment, the process for the preparation for the pharmaceutical composition of diclofenac and eletriptan comprises the following steps:
a. Mixing microcrystalline cellulose and lactose monohydrate
b. Adding diclofenac or pharmaceutically acceptable salt thereof and eletriptan or pharmaceutically acceptable salt thereof to the powder mixture and mixing
c. Adding povidone (polyvinylpyrrolidone) and croscarmellose sodium (cross-linked carboxymethyl cellulose sodium) to the powder mixture
d. Sieving colloidal silicon dioxide with a 0.6 mm sieving mesh
e. Adding sieved colloidal silicon dioxide to the powder mixture and mixing
f. Adding magnesium stearate to the mixture and mixing
g. Filling the powder mixture into capsules.

### Example 1: Capsule

| **Ingredients** | **% by weight** |
|---|---|
| Diclofenac potassium | 5.0-40.0 |
| Eletriptan hydrobromide | 5.0-40.0 |
| Microcrystalline cellulose | 5.0-50.0 |
| Lactose monohydrate | 5.0-50.0 |
| Povidone K30 | 0.1-5.0 |
| Croscarmellose sodium | 0.1-10.0 |
| Colloidal silicon dioxide | 0.1-10.0 |
| Magnesium stearate | 0.1-10.0 |
| **Total** | 100.0 |

### Production process:

a. Mixing microcrystalline cellulose and lactose monohydrate
b. Adding diclofenac and eletriptan hydrobromide to the powder mixture and mixing
c. Adding Povidone K30 and croscarmellose sodium (cross-linked carboxymethyl cellulose sodium) to the powder mixture
d. Sieving colloidal silicon dioxide with a 0.6 mm sieving mesh
e. Adding sieved colloidal silicon dioxide to the powder mixture and mixing
f. Adding magnesium stearate to the mixture and mixing
g. Filling the powder mixture into capsules.

### Example 2: Capsule

| **Ingredients** | **% by weight** |
|---|---|
| Diclofenac potassium | 10.0-20.0 |
| Eletriptan hydrobromide | 10.0-20.0 |
| Microcrystalline cellulose | 30.0-40.0 |
| Lactose monohydrate | 20.0-30.0 |
| Povidone K30 | 0.1-1.0 |
| Croscarmellose sodium | 1.0-10.0 |
| Colloidal silicon dioxide | 1.0-5.0 |
| Magnesium stearate | 1.0-5.0 |
| **Total** | 100.0 |

### Production process:

a. Mixing microcrystalline cellulose and lactose monohydrate
b. Adding diclofenac and eletriptan hydrobromide to the powder mixture and mixing
c. Adding Povidone (polyvinylpyrrolidone) and croscarmellose sodium (cross-linked carboxymethyl cellulose sodium) to the powder mixture
d. Sieving colloidal silicon dioxide with a 0.6 mm sieving mesh
e. Adding sieved colloidal silicon dioxide to the powder mixture and mixing
f. Adding magnesium stearate to the mixture and mixing
g. Filling the powder mixture into capsules.

## Claims

1. A pharmaceutical composition comprising diclofenac or a pharmaceutically acceptable salt thereof in combination with eletriptan or a pharmaceutically acceptable salt thereof and at least one pharmaceutically acceptable excipient.

2. The pharmaceutical composition according to claim 1, wherein diclofenac or a pharmaceutically acceptable salt thereof is present in an amount of between %5 and %40 by weight of total formulation, eletriptan or a pharmaceutically acceptable salt thereof is present in an amount of between %5 and %40 by weight of total formulation.

3. The pharmaceutical composition according to claim 2, wherein the weight ratio of diclofenac or a pharmaceutically acceptable salt thereof to eletriptan or a pharmaceutically acceptable salt thereof is between 0.2 and 5.0, preferably between 0.3 and 3.0, more preferably between 0.5 and 2.0.

4. The pharmaceutical composition according to any of the preceding claims, wherein the dosage form is in the form of a tablet, mini tablet, bilayer tablet, trilayer tablet, multilayer tablet, capsule, tablet in tablet, an inlay tablet, injectable preparate, suspension, syrup, sachet, ointment, cream or gel.

5. The pharmaceutical composition according to claim 4, wherein the dosage form is in the form of a capsule.

6. The pharmaceutical composition according to claim 1, wherein the pharmaceutically acceptable excipient is selected from a group comprising comprising disintegrants, fillers, binders, lubricants, glidants or mixtures thereof.

7. The pharmaceutical composition according to claim 6, wherein the disintegrant is selected from a group comprising cross-linked carboxymethyl cellulose sodium (croscarmellose sodium), starch, crospovidone (cross-linked polyvinil pyrrolidone), poloxomer, low-substituted hydroxypropyl cellulose, pregelatinized starch, sodium carboxymethyl cellulose, calcium carboxymethyl cellulose, carboxymethyl cellulose, docusate sodium, guar gum, , polyacryline potassium, sodium alginate, corn starch, sodium starch glycolate, alginic acid, alginates, ion-exchange resins, magnesium aluminium silica, sodium dodesyl sulphate, sodium glycine carbonate, sodium lauryl sulphate or mixtures thereof.

8. The pharmaceutical composition according to claim 7, preferably the disintegrant is croscarmellose sodium.

9. The pharmaceutical composition according to claim 7, the disintegrant is present in an amount of between 0.1% and 10% by weight of total formulation.

10. The pharmaceutical composition according to any of the preceding claims, comprising;
a. 5.00 to 40.00 % diclofenac or a pharmaceutically acceptable salt thereof
b. 5.00 to 40.00 % eletriptan or a pharmaceutically acceptable salt thereof
c. 5.00 to 50.00 % microcrystalline cellulose
d. 5.00 to 50.00 % lactose monohydrate
e. 0.1 to 5.00 % povidone (polyvinylpyrrolidone)
f. 0.1 to 10.00 % croscarmellose sodium
g. 0.1 to 10.00 % colloidal silicon dioxide
h. 0.1 to 10.00 % magnesium stearate
by weight of the total formulation

11. The process for preparation of the pharmaceutical composition according to claim 10, wherein the process comprising the following steps:
a. Mixing microcrystalline cellulose and lactose monohydrate
b. Adding diclofenac and eletriptan hydrobromide to the powder mixture and mixing
c. Adding povidone and croscarmellose sodium to the powder mixture
d. Sieving colloidal silicon dioxide with a 0.6 mm sieving mesh
e. Adding sieved colloidal silicon dioxide to the powder mixture and mixing
f. Adding magnesium stearate to the mixture and mixing
g. Filling the powder mixture into capsules.
